# EUROPÄISCHE PATENTSCHRIFT

(11) **EP 0 423 262 B1**
(45) Veröffentlichungstag und Bekanntmachung des Hinweises auf die Patenterteilung: **19.01.1994**
(21) Anmeldenummer: 90904324.2
(22) Anmeldetag: 09.03.1990
(51) Int. Cl.: A61K 35/78

(54) **PROTEINFRAKTION AUS DEM PHOTOSYNTHESEAPPARAT VON PFLANZEN ZUR ANWENDUNG ALS WIRKSTOFF ZUR SELEKTIVEN AUFLÖSUNG VON KREBSZELLEN UND ZUR NACHBEHANDLUNG VON NARBEN, KELOIDEN UND ENTZÜNDLICHEN PROZESSEN SOWIE DIESE ENTHALTENDE PHARMAZEUTISCHE ERZEUGNISSE**
PROTEIN FRACTION FROM THE PHOTOSYNTHESIS APPARATUS OF PLANTS FOR USE AS AN ACTIVE SUBSTANCE IN THE SELECTIVE DESTRUCTION OF CANCER CELLS AND THE POST-TREATMENT OF SCARS, KELOIDS AND INFLAMMATORY PROCESSES AND PHARMACEUTICAL PRODUCTS CONTAINING IT
FRACTION PROTEINIQUE DE L'APPAREIL DE PHOTOSYNTHESE VEGETAL UTILE COMME SUBSTANCE ACTIVE DE DISSOLUTION SELECTIVE DE CELLULES CANCEREUSES ET POUR LE TRAITEMENT ULTERIEUR DE CICATRICES, DE CHELOIDES ET DE PROCESSUS INFLAMMATOIRES; SUBSTANCES PHARMACEUTIQUES CONTENANT CETTE FRACTION PROTEINIQUE

(30) Priorität: 10.03.1989 DE 3907822
(43) Veröffentlichungstag der Anmeldung: 24.04.1991
(73) Patentinhaber: LÖFFLMANN, Adolf, D-81379 München (DE)
(72) Erfinder: LÖFFLMANN, Adolf, D-81379 München (DE)
(74) Vertreter: Andrae, Steffen, Dr.
(86) Internationale Anmeldenummer: EP9000388
(87) Internationale Veröffentlichungsnummer: WO9010452

(56) Entgegenhaltungen:
- EP-A- 0 267 463
- DE-A- 1 617 391
- Carlsberg Res. Commun., vol. 46, 1981, (Copenhagen), E.A.H. Landis et al., pp. 127-242

## Beschreibung

Die Erfindung betrifft die auf ein neuartiges medizinischwissenschaftliches Konzept zurückgehende Anwendung bestimmter, nachfolgend näher erläuterter Substanzen als Arzneimittelwirkstoff, insbesondere zur selektiven Auflösung (Abtötung) von Krebszellen (malignen Tumorzellen), jedoch auch generell zur Bekämpfung entzündlicher Prozesse sowie zur Behandlung hypertropher Narben und Keloide.

Die vorliegende Erfindung geht zurück auf Überlegungen, daß die bisherigen wissenschaftlichen Ansätze zum Verständnis und zur Bekämpfung von Krebs möglicherweise auch deshalb so unbefriedigend geblieben sind, weil die dahinterstehende Vorstellung, daß das Krebsgeschehen ursächlich mit dem Zellkern verknüpft ist, den Blick für grundsätzlich neue erfolgversprechende Konzepte verstellt hat. Krebszellen unterscheiden sich von gesunden Zellen in einer Reihe von Eigenschaften, und eine erfolgreiche Krebstherapie sollte nach Möglichkeit an diesen, Krebszellen von gesunden Zellen unterscheidenden Eigenschaften ansetzen, um einen selektiv nur zu Krebszellen führenden bzw. selektiv nur Krebszellen schädigenden Therapieweg wählen zu können. Zahlreiche bekannte Verfahren der Krebstherapie erfüllen jedoch diese Forderungen nicht, da sie wie z.B. die meisten Cytostatika und Strahlen nicht selektiv auf Krebsgewebe einwirken, sondern auch normales gesundes Gewebe schädigen. Ein aus der Literatur bekanntes Verfahren zur Zerstörung von Tumoren ist beschrieben in Nachr. Chem. Tech. Lab. 33 (1985), Nr. 7 "Lokalisierung und Therapie von Tumoren mit Porphyrinen", von H. Vandenbergh und P. Cornaz. Bei diesem Verfahren werden Injektionen eines Hämatoporphyrin-Derivats mit einer Rotlichtbestrahlung kombiniert.

Die vorliegende Erfindung geht aus von der bekannten Beobachtung, daß Krebszellen sich von gesunden Zellen unter anderem dadurch unterscheiden, daß sie einen "Sauerstoffmangel-Stoffwechsel" aufweisen, für den das Auftreten der aeroben Glycolyse kennzeichnend ist. Ausgehend von diesem medizinischen Faktum ging die Überlegung dahin, daß eine Therapie, die gezielt an der von gesunden Zellen unterschiedlichen Zellatmung von Krebszellen ansetzt, diese selektiv schädigen kann, ohne gleichzeitig gesunde Zellen anzugreifen. Bei der Weiterverfolgung dieses Konzepts erwies es sich dann jedoch, daß für eine erfolgreiche Therapie weitere Besonderheiten von Krebszellen gegenüber normalen Zellen berücksichtigen werden müssen und daß hilfsreiche Modellüberlegungen dann angestellt werden können, wenn man die Krebszelle als eine Zelle betrachtet, die insgesamt embryonale Verhaltensweisen (embryonale Kodierung) nachahmt. Zu den Eigenschaften, die Krebszellen ähnlich wie embryonale Zellen aufweisen, gehört jedoch nicht nur der Sauerstoffmangelbetrieb des Zellstoffwechsels, sondern auch eine Proteasenaktivität, die den Krebszellen wie embryonalen Zellen die Fähigkeit zum "Andauen" von Körpergewebe (mütterliches Uterusgewebe bzw. gesundes Gewebe des Krebskranken) verleiht.

Aus diesen Grundüberlegungen wurde eine neuartige Krebstherapie entwickelt, deren Wirksamkeit im Tierversuch bestätigt werden konnte und die sich auch schon in Einzelfällen klinisch als erfolgreich erwies. Diese neue Krebstherapie besteht in einem Verfahren zur selektiven Auflösung von Krebszellen im lebenden Warmblüterorganismus durch Verabreichung in Kombination, und zwar entweder in enger zeitlicher Aufeinanderfolge oder in Form eines Gemischs, per Injektion oder Infusion von einer Proteinfraktion mit Sauerstoffaktivität aus dem Photosyntheseapparat von Pflanzen oder dessen Vorstufen und einem Proteingemisch aus embryonalem Gewebe oder maternalem Uterusgewebe von Plazentatieren, wobei die Verabreichung in Form steriler, isotonischer und endotoxinfreier Lösungen erfolgt.

Die krebszellwachstumshemmende Wirkung von bestimmten Pflanzenextrakten wurde bereits verschiedentlich beschrieben (vgl. z.B. Planta Med. 1985, (6), 538-539; DE-AS 16 17 391). Bei diesen Veröffentlichungen geht es jedoch um Wirkungen, die als Eindämmung und Verzögerung des Tumorwachstums beschreibbar sind, jedoch nicht um eine Tumorauflösung. In der DE-OS 29 20 631 wird die Verwendung eines Preßsafts fleischfressender Pflanzen, insbesondere der Venusfliegenfalle, beansprucht, und es wird eine Auflösung von Tumoren geltend gemacht. Der Erfolg der beschriebenen Behandlung ist jedoch umstritten, und die Verwendung des genannten Preßsafts wegen eines hohen Endotoxingehalts bedenklich.

Es ist ferner bekannt, daß Extrakte aus der Plazenta, insbesondere Rinderplazenta, eine tumorspezifische Inhibitionswirkung aufweisen können (Exp. Path., Bd. 8, Seite 205-212 sowie Bd. 9, Seite 354-360; Österreichische Zeitschrift für Onkologie, 1974, Heft 2, Seite 31-37 sowie ibid, 1977, Heft 2-3, Seite 42-46; EP-A-136 093). Derartige Extrakte werden als das Wachstum der Tumorzellen hemmend beschrieben, es wird jedoch nicht von einer Krebstumorauflösung in vivo berichtet.

Die Kombination einer Verwendung von Pflanzenextrakten mit einer aus embryonalem Gewebe gewonnenen Extraktfraktion wurde bisher noch nicht beschrieben. Die Möglichkeit einer Tumorauflösung mit einer Wirkstoffkombination der genannten Art kann daher selbstverständlich dem Stand der Technik ebenfalls nicht entnommen werden. Bei der Schaffung der vorliegenden Erfindung zeigte es sich, daß eine Kombination zweier Proteinfraktionen bzw. Proteingemische unterschiedlichen Ursprungs, die nachfolgend noch näher beschrieben werden, im Sinne einer Krebszellauflösung selektiv wirksam sind, während bei einer alleinigen Anwendung einer der beiden genannten Fraktionen keine vergleichbare Wirkung erhalten wird.

Die Patentansprüche der vorliegenden Anmeldung sollen die dem Patentrecht zugänglichen Aspekte der neuartigen Krebstherapie abdecken, die mit der vorliegenden Anmeldung offenbart wird und die eine Lösung für die Aufgabe darstellt, eine Therapie zu schaffen, die eine selektive Auflösung von Krebszellen mit Medikamenten ermöglicht, die für den gesunden Körper und gesunde Zellen völlig unschädlich sind.

Die Erfindung betrifft somit einmal eine Proteinfraktion mit Sauerstoffaktivität aus dem Photosyntheseapparat von Pflanzen oder aus dessen Vorstufen, die erhältlich ist durch an sich bekannte Isolierung von Chloroplasten und/oder ihren Vorstufen aus autotrophen Pflanzen oder autotrophen niederen Lebewesen, Waschen der isolierten Chloroplastenfraktion, Behandeln der isolierten und gewaschenen Chloroplasten mit einer hypotonischen Äthanollösung, Abtrennen des Sediments, Auftrennen der alkoholischen Lösung durch trägerfreie Ablenkungselektrophorese und Gewinnung einer gelblichen, Sauerstoff freisetzenden Proteinfraktion sowie ggf. anschließendes Aufkonzentrieren dieser Fraktion durch Ultrafiltration, Dialyse des Ultrafiltrationsretentats gegen Wasser sowie Gefriertrocknen, zur Anwendung als in Kombination mit einem aus embryonalem Gewebe oder aus maternalem Uterusgewebe von Plazentatieren gewonnenen Proteingemisch per Injektion oder Infusion zu verabreichender pharmazeutischer Wirkstoff zur selektiven Auflösung von Krebszellen im lebenden Warmblüterorganismus.

Die Erfindung betrifft ferner auch noch eine derartige Proteinfraktion zur Anwendung zur Nachbehandlung von Narben, Keloiden sowie von entzündlichen Prozessen, die auf die Einwirkung ionisierender Strahlen zurückgeht.

Die Erfindung betrifft ferner pharmazeutische Erzeugnisse, enthaltend (a) eine eine Sauerstoffaktivität aufweisende Proteinfraktion aus dem Photosyntheseapparat von Pflanzen oder aus dessen Vorstufen, die erhältlich ist durch an sich bekannte Isolierung von Chloroplasten und/oder ihren Vorstufen aus autotrophen Pflanzen oder autotrophen niederen Lebewesen, Waschen der isolierten Chloroplastenfraktion, Behandeln der isolierten und gewaschenen Chloroplasten mit einer hypotonischen Äthanollösung, Abtrennen des Sediments, Auftrennen der alkoholischen Lösung durch trägerfreie Ablenkungselektrophorese und Gewinnung einer gelblichen, Sauerstoff freisetzenden Proteinfraktion sowie ggf. anschließendes Aufkonzentrieren dieser Fraktion durch Ultrafiltration, Dialyse des Ultrafiltrationsretentats gegen Wasser sowie Gefriertrocknen,und (b) ein aus embryonalem Gewebe oder aus maternalem Uterusgewebe von Plazentatieren gewonnenes Proteingemisch als per Injektion oder Infusion im Gemisch oder in unmittelbarer zeitlicher Aufeinanderfolge zu verabreichendes Kombinationspräparat zur selektiven Auflösung von Krebszellen im lebenden Warmblüter-organismus.

Die Erfindung betrifft somit einmal die Verwendung einer bisher nach diesseitiger Kenntnis noch nicht als Arzneimittel verwendeten Substanzklasse, nämlich die Verwendung von Sauerstoffaktivität (Freisetzung von Sauerstoff aus Wasser bei Licht- und/oder Wärmeeinwirkung) zeigenden, auf die beschriebene Weise erhältlichen Proteinfraktionen aus dem Photosyntheseapparat frischer oder gegebenenfalls getrockneter Pflanzen oder niederer autotropher Lebewesen als Wirkstoff eines per Injektion oder Infusion in Kombination mit einem aus embryonalen Zellen oder maternellem Uterusgewebe gewonnenen Proteingemisch verabreichbaren Arzneimittels zur selektiven Auflösung (Zerstörung) von Krebszellen.

Der Grund für die Verwendung einer Proteinfraktion aus dem Photosyntheseapparat autotropher Pflanzen oder autotropher niederer Lebewesen liegt vereinfacht gesagt darin, daß bei der Photosynthese der Pflanze eine Reihe von Redox- und Elektronentransportvorgängen in umgekehrter Richtung verlaufen wie bei der Zellatmung. Diese findet bei höheren Lebewesen in den Mitochondrien statt. In den Lehrbüchern und der Fachliteratur sind die für die Zellatmung charakteristischen Elektronenübertragungs-Teilschritte zwischen bestimmten Enzymen der Atmungskette genau beschrieben. Bei einer im Sauerstoffmangelbetrieb funktionierenden Zelle wie der Krebszelle sind die Mitochondrien weitgehend blockiert. Das kann vermutlich darauf zurückgeführt werden, daß Elektronen liefernde Fremdsubstanzen ein Enzym der Atmungskette reduktiv absättigen (Fe³⁺ → Fe²⁺) und damit für die Elektronenaufnahme aus der Atmungskette blockieren. Indem einer solchen blockierten, im Sauerstoffmangelbetrieb funktionierenden Zelle (einer Krebszelle) eine den Effekt der Blockierung durch ein überschüssiges Elektron rückgängig machende Substanz angeboten wird, kann der Stoffwechsel einer solchen Zelle zum Entgleisen gebracht werden, mit der Folge, daß sie abstirbt. Als eine im obigen Sinne wirksame Substanzklasse wurden die Enzyme des Photosyntheseapparats autotropher Pflanzen erkannt. Diese Substanzen sind in den Chloroplasten grüner Pflanzen lokalisiert, und dabei insbesondere in der Thylakoidmembran dieser Chloroplasten.

Die Gewinnung und geeignete Aufbereitung einer derartigen geeigneten Proteinfraktion aus dem Photosyntheseapparat von Pflanzen wird nachfolgend noch genauer beschrieben. Es zeigte sich, daß die wirksame Proteinfraktion ein Enzym darstellt oder enthält, das bei Zimmertemperatur aus H₂O Sauerstoff abspaltet (= Sauerstoffaktivität aufweist). Die Isolierung und Charakterisierung derartiger Proteinfraktionen ist für bestimmte Einzelfälle auch bereits in der Literatur beschrieben. Es kann hierzu verwiesen werden auf Carlsberg Res. Commun. Vol. 45, Seite 167-176, 1980; Carlsberg Res. Commun. Vol. 46, Seite 227-242, 1981; Proc. Natl. Acad. Sci. USA, Vol. 77, No. 2, Seite 957-959, 1980. Es wird angenommen, daß die auf die nachfolgend beschriebene Weise erhaltene und im Rahmen der vorliegenden Erfindung verwendete Proteinfraktion in der Wirkung sowie gegebenenfalls auch ihrer Natur nach den in den genannten Arbeiten beschriebenen Proteinen entspricht. Es sei an dieser Stelle bereits angemerkt, daß ungeachtet der Tatsache, daß die experimentellen Befunde unter Verwendung einer auf eine konkret beschriebene Weise aus einer bestimmten Pflanze (Equisetum arvense = Ackerschachtelhalm oder Zinnkraut) erhaltenen Proteinfraktion erhalten wurden, dieses konkrete Verfahren und auch das konkrete pflanzliche Ausgangsmaterial nicht kritisch sein dürften, sondern daß ein in gleichem Sinne wirksames Material aus den meisten oder allen grünen Pflanzen isolierbar sein müßte, da seine Rolle im pflanzlichen Photosyntheseapparat von Bedeutung ist, jedoch nicht seine Herkunft aus einer bestimmten Pflanze.

Beim Versuch der alleinigen Verwendung der Proteinfraktion aus dem Photosyntheseapparat autotropher Pflanzen zeigte sich jedoch, daß diese zwar bei ihrer Verabreichung per Injektion (intravenös, intramuskulär, subkutan) physiologisch aktiv war, jedoch Krebszellen nicht im erwünschten Sinne schädigte. Es zeigte sich, daß bei der Injektion der genannten Substanz ein normales Gewebe Reaktionen zeigte, die sich als Entzündungs- hemmung im weiteren Sinne beschreiben lassen. So wurde ein ausgeprägter Effekt bei der Injektion nach einer Strahlentherapie beobachtet, indem die Entzündungserscheinungen in dem strahlengeschädigten normalen Gewebe rasch zum Abklingen gebracht wurden.

Die insignifikante Wirkung auf Krebszellen konnte dann damit erklärt werden, daß die Krebszellen aufgrund der erhöhten Proteasenaktivität (z.B. Kollagenase IV) das Eindringen des proteinischen pflanzlichen Enzyms verhinderten, vermutlich unter dessen Zerstörung. Unter fruchtbarer Anwendung der Modellvorstellung "Krebszelle = embryonal kodierte Zelle" gelang es jedoch, dieses Problem dadurch zu überwinden, daß zusammen mit der erwähnten Proteinfraktion aus Chloroplasten ein Extrakt aus embryonalem und foetalem Gewebe von Plazentatieren injiziert wurde, dessen Verwendung in Kombination mit der bereits beschriebenen Proteinfraktion aus dem Photosyntheseapparat von Pflanzen ein wesentliches Element der vorliegenden Erfindung darstellt. Es sei an dieser Stelle darauf hingewiesen, daß der Begriff "Plazentatiere" im weitesten Sinne zu verstehen ist und daß auch aus derartigem Gewebe humanen Ursprungs die erforderlichen Wirksubstanzen gewinnbar sein müßten. Es wird angenommen, daß der Effekt der zusätzlichen Injektion des Proteinmischungs-Extrakts aus embryonalem Gewebe darin besteht, die Proteasen der Krebszellen abzusättigen, zu blockieren oder in anderer Weise zu entaktivieren, so daß nunmehr die Proteine aus dem Photosyntheseapparat der Pflanzen in die Krebszelle gelangen und dort ihre Wirkung entfalten können.

Diese Erklärung der Wirkung der Injektion des Embryonalgewebeextrakts bedeutet gleichzeitig, daß dieser Extrakt in allen Fällen vorteilhaft zur Anwendung kommen kann, in denen versucht wird, Krebszellen mit Hilfe pflanzlicher Materialien proteinischer Natur zu beeinflussen, bei denen jedoch die aufgrund der in vitro-Untersuchungen erwarteten Ergebnisse durch die klinische Praxis nicht oder nicht mit hinreichender Deutlichkeit verifiziert werden konnten.

Nachfolgend wird die erfindungsgemäße neuartige Krebstherapie durch detaillierte Beschreibung der Herstellung der verwendeten Materialien, der Ergebnisse der Tierversuche sowie der klinischen Befunde näher erläutert.

Es sei dabei an dieser Stelle darauf hingewiesen, daß die beschriebenen Herstellungsverfahren zwar einen Weg zur Gewinnung verwendbarer wirksamer Substanzen beschreiben, daß derartige oder im Sinne des Konzepts der vorliegenden Erfindung gleichwertige Substanzen auch nach mehr oder weniger stark modifizierten derartigen Verfahren erhältlich sein dürften.

### I. Herstellung und Charakterisierung einer Sauerstoff freisetzenden Proteinfraktion aus Chloroplasten von Equisetum arvense.

Ausgehend von frischen Stengeln und Blättern von Equisetum arvense (100 g) werden nach einem Standardverfahren, wie es beispielsweise beschrieben ist in dem Buch "Praktikum zur Stoffwechselphysiologie der Pflanzen", Georg Thieme Verlag Stuttgart, New York, 1983, 2. Auflage, Seite 66-68, intakte Chloroplasten isoliert. Dazu zerkleinert man die vorzerkleinerten Pflanzenteile in einem Mixer in Gegenwart einer Lösung, die aus 0,33 M Saccharose, 0,01 M Natriumdiphosphat, 5 mM MgCl₂ in einem Liter destilliertem Wasser unter Einstellung des pH mit Salzsäure auf 3 hergestellt wurde. Das erhaltene Homogenisat wurde ausgepreßt, und der Preßsaft zur Gewinnung einer Chloroplasten-Fraktion zentrifugiert. Der Überstand wurde verworfen, und die Chloroplasten-Fraktion wurde mit einer geeigneten sauren (pH ca. 3,5) isotonischen Lösung gewaschen.

Zum Aufbrechen der Chloroplasten wurde die Chloroplasten-Fraktion anschließend mit einer hypotonischen sauren (pH 3,5) Äthanollösung, deren Äthanolgehalt vorzugsweise im Bereich von 30 bis 70 Volumen-%, vorzugsweise im Bereich von 35 bis 50 Volumen-% liegt, behandelt. Es wurde eine gelbe Lösung erhalten, die von den als Sediment anfallenden Chloroplastenhüllen abgetrennt wurde.

Diese Lösung kann als eine Art Rohprodukt durch stufenweise Sterilfiltration über Filter mit einer Porenweite von 8 µm, 0,45 µm und 0,2 µm direkt aufgereinigt und anschließend gefriergetrocknet werden.

Vorzugsweise wird die gelbe äthanolische Lösung jedoch über trägerfreie Ablenkungselektrophorese (vgl. Kurt Hannig in: Jahrbuch 1968 der Max-Planck-Gesellschaft zur Förderung der Wissenschaften e.V. oder Z. Anal. Chem., 181, 244 (1961)) aufgetrennt, und es werden diejenigen Fraktionen gewonnen, die eine gelbliche Färbung aufweisen. Diese Fraktionen werden vereinigt.

Die das gewünschte Protein mit einer Sauerstoff aus Wasser freisetzenden Wirkung enthaltende Fraktion wird vorzugsweise unter Verwendung einer Ultrafiltrations-Membran mit einer Schnittzone im Bereich von 5000 bis 10 000 d auf etwa das 0,3- bis 0,7fache ihres Ausgangsvolumens konzentriert, wonach das als Retentat erhaltene Konzentrat zur Entfernung des Puffers gegen angesäuertes reines Wasser (pH ca. 3,5) dialysiert wird. Das durch Dialyse gereinigte Produkt wird dann anschließend wie beschrieben einer Sterilfiltration unterzogen und gefriergetrocknet.

Vorzugsweise werden jedoch vor der Gefriertrocknung noch gegebenenfalls in der Lösung vorhandene Endotoxine auf adsorbtivem Wege unter Verwendung geeigneter Adsorptionsmittel entfernt. Geeignete Adsorptionsmittel sind beispielsweise Q-Sepharose, Polymyxin B-Sepharose und Aktivkohle. Von diesen Adsorbenzien ist Aktivkohle weniger bevorzugt, da bei ihrer Verwendung auch erhebliche Verluste des gewünschten proteinischen Materials beobachtet werden.

Zur Bereitung einer injizierbaren Lösung wird das gefriergetrocknete Produkt in physiologischer Kochsalzlösung aufgenommen, und zwar in Mengen von etwa 1 bis 28 mg/ml physiologischer Kochsalzlösung. Es ist dabei darauf hinzuweisen, daß sich gezeigt hat, daß das gewünschte Protein auch in einer alkoholischen Lösung enthalten ist, die durch direkte Behandlung von zerkleinerten getrockneten Blättern und Stengeln von Equisetum arvense mit einer hypotonischen Äthanollösung erhalten wird. Auch eine solche Lösung kann im beschriebenen Sinne aufgereinigt, steril filtriert und gefriergetrocknet werden. Aufgrund der Verwendung einer hypotonischen sauren Alkohollösung als Extraktionsmedium besteht in der Regel keine Gefahr einer mikrobiologischen Verunreinigung des erhaltenen Extrakts.

Die aus Equiseum arvense gewonnene Proteinfraktion wird durch SDS-Gradienten-Gelelektrophorese (PAA 4/30 Gel der Firma Pharmacia Fine Chemicals AB; Probenaufbereitung und Durchführung der Elektrophorese nach Herstellervorschrift) in Gegenwart eines Markers aufgetrennt. Es wurde eine Hauptbande mit einem Molekulargewicht von 67000 ± 10000 d erhalten. Schwächere Nebenbanden waren bei Molekulargewichten von 83500 ± 10000 und 47000 ± 10000 zu erkennen. Die durch isoelektrische Fokussierung bestimmten isoelektrischen Punkte lagen bei 4,1 (Hauptfraktion; zwei eng beieinanderliegende Banden), sowie 4,7 ± 0,3 und 5,0 ± 0,3 (schwächere Banden). Es wurde ein Marker für den pH-Bereich von 4,7 bis 10,6 verwendet.

Die Lösung des Proteins ist in höheren Konzentrationen gelb gefärbt und in Gegenwart von Protonenakzeptoren bzw. im neutralen bis schwach basischen Milieu bei Lichtzutritt und Erwärmung bzw. unter IR-Komponenten enthaltendem Licht unter Freisetzung von Sauerstoff aus Wasser bei gleichzeitiger Absenkung des pH-Werts zersetzlich. Bei der Gewinnung der Proteinfraktion werden daher vorsorglich saure pH-Werte eingehalten. Es hat sich ferner als vorteilhaft erwiesen, alle Schritte im Zusammenhang mit der Isolierung der Proteinfraktion bei niedrigen Temperaturen, vorteilhaft bei +4°C, durchgeführt. Wenn der Puffer durch Dialyse gegen entionisiertes Wasser entfernt wurde, wird eine gegen Lichteinwirkung instabile Lösung erhalten, die jedoch unter Lichtausschluß (gefärbte2 Gefäße) ohne Probleme gefriergetrocknet werden kann.

### II. Gewinnung eines injizierbaren Extrakts (Proteingemisch) aus embryonalem Gewebe oder maternalem Uterusgewebe.

Aus dem Schlachthof bezogene Fruchtsäcke von Kälbern oder Schafen werden unter sterilen Bedingungen in einer Laminar-Flow-Kammer geöffnet, und die entnommenen Plazenten und Nabelschnüre sowie gegebenenfalls Embryonen und Foeten werden sofort auf unter -20°C, insbesondere -26°C, schockgefroren.

Als Ausgangsmaterial kann stattdessen aber auch maternales Uterusgewebe trächtiger Tiere, beispielsweise trächtiger Schafe und Rinder, eingesetzt und analog verarbeitet werden.

400 bis 500 g dieser schockgefrorenen Proben von Embryonalgewebe werden unter Zusatz von einem Liter von schwach saurem bis schwach alkalischem wäßrigen Äthanol (35 Volumen-%) in einen Mixer gegeben und in Gegenwart des Extraktionsmittels homogenisiert, wobei die Temperatur auf +4°C gehalten wird. Das erhaltene Homogenisat wird zur Verbesserung der Sedimentierbarkeit bzw. Abtrennbarkeit sedimentierer Bestandteile vorzugsweise bei +4°C einige Tage in Gegenwart von Glasperlen geschüttelt, wonach die sedimentierbaren Bestandteile durch Abzentrifugieren oder Filtration unter sterilen Bedingungen abgetrennt werden.

Das erhaltene Filtrat oder der Überstand wird anschließend einer vorzugsweise mehrstufigen Sterilfiltration unterzogen (Filter-Porenweiten 8 µm; 0,45 µm; 0,2 µm), wobei die Temperatur der Lösungen stets bei +4°C gehalten wird. Das Filtrat der letzten Filtration wird anschließend gefriergetrocknet.

Vorzugsweise erfolgt vor dem Gefriertrocknen, wie im Falle der unter I. beschriebenen Proteinfraktion, eine vorsorgliche adsorptive Entfernung von Endotoxinen.

Durch Aufnehmen in einem wäßrigen Medium (etwa 1 bis 28 mg/ml wäßriges Medium bzw. physiologische Kochsalzlösung) unter isotonischen Bedingungen kann aus dem gefriergetrockneten Produkt eine injizierbare Lösung bereitet werden.

SDS-Polyacrylamid-Gelelektrophorese (PAA 4/30 der Firma Pharmacia Fine Chemicals AB; Auftrennung nach Firmenvorschrift) lieferte eine Hauptbande bei 62000 ± 10000 d. Nebenbanden waren bei 43000 ± 10000 sowie 13800 ± 5000 zu erkennen. Bei der isoelektrischen Fokussierung wurden Gemischbestandteile mit isoelektrischen Punkten bei 4,7 ± 0,3 (Hauptbande) sowie Nebenbanden bei 4,1 (zwei eng beieinanderliegende Banden) und 4,8 bis 5,1 (drei benachbarte Banden) gefunden.

Bei der Celluloseacetatfolienelektrophorese lief der aus Plazenta und Nabelschnur gewonnene Extrakt im Bereich der Albuminfraktion sowie der α-Globulinfraktion (70 % Albumin; 27,6 % α₁-Globulin; 2,4 % α₂-Globulin).

Zur Gewinnung des Extrakts aus embryonalem Gewebe (Plazenta/Nabelschnüre/Foeten) kann ein embryonales Gewebe jedes beliebigen Reifezustands verwendet werden.

### III. In vivo-Untersuchungen an Ratten.

In Wistar-Ratten wurden subkutan durch einmalige Verabreichung von 20 mg 20-Methylcholanthren Fibrosarkome erzeugt. Sobald diese auf eine für die beabsichtigten Versuche geeignet erscheinende Größe herangewaschsen waren, wurde die Behandlung mit der aus Equisetum arvense gewonnenen Proteinfraktion (abgekürzt MDA) sowie dem als Proteinaseinhibitor wirkenden Embryonalgewebeextrakt (abgekürzt PI) aufgenommen. Beide Substanzen wurden im Gemisch bzw. unmittelbar nacheinander subkutan bzw. intratumoral injiziert. Die Wirkung dieser Behandlung wurde kontinuierlich dadurch verfolgt, daß die Menge des von der Ratte bei gleichbleibender Futtermenge und unbegrenzter Flüssigkeitsaufnahme ausgeschiedenen Urins (ml/24 h) sowie die Menge an ausgeschiedener Harnsäure (mg/24 h) verfolgt wurden. Die Menge der ausgeschiedenen Harnsäure wird als direktes Maß für die Zerstörung der Tumorzellen genommen, da für die Purinbasen Adenin und Guanin aus der DNA nur ein einziger biochemischer Abbauweg existiert, der zum Endprodukt Harnsäure führt, die dann im Urin ausgeschieden wird. Die gemessene Menge an täglich ausgeschiedener Harnsäure stellt damit ein Maß für die Menge des abgebauten Adenins und Guanins dar, und da diese nur aus der DNA zerstörter Zellen stammen können, auch ein Maß für die Zerstörung der DNA von Tumorzellen und damit der gesamten Tumorzellen.

Nach Beendigung der Versuche wurden die getöteten Versuchstiere einer mikroskopischen Gewebsuntersuchung (Tumorgewebe, Gewebe der inneren Organe) unterzogen.

Bei einer ersten Ratte, deren Tumor bei Behandlungsbeginn einen Außendurchmesser von 0,8 cm aufwies, führte die tägliche subkutane Injektion von 100 mg MDA in Verbindung mit einer Injektion von wöchentlich 12 mg PI dazu, daß das chemisch induzierte subkutane Fibrosarkom verschwand. Während der Behandlung wurde eine deutlich über dem Normalbereich liegende tägliche Harnsäureausscheidung beobachtet. Dabei wurde als Normalwert für die tägliche Harnsäureausscheidung gesunder Tiere ein Wert der Größenordnung von 0,4 bis 0,6 mg/24 h ermittelt, für Tiere mit Tumoren etwas erhöhte Werte im Bereich von 0,5 bis 1,5 mg/24 h (jeweils konstante Futtermenge, unbeschränkte Flüssigkeitszufuhr).

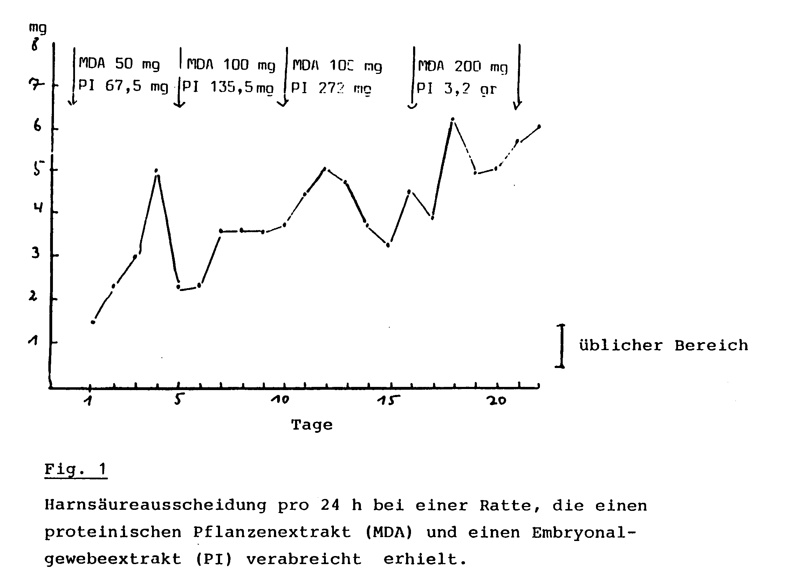

Nach dem geschilderten erfolgreichen Vorversuch wurde eine weitere Ratte mit einem chemisch induzierten Fibrosarkom (äußerer Tumordurchmesser bei Behandlungsbeginn ca. 3 x 3,5 cm) nach dem sich aus **Figur 1** ergebenden Injektionsschema mit steigenden, jeweils gemeinsam verabreichten Dosen MDA und PI behandelt, die subkutan und intratumoral injiziert wurden. Die tägliche Harnsäureausscheidung stieg in der Folge der intratumoralen Injektionen bis auf Werte von 5 und 6,12 mg/24 h, d.h. bis auf das 10fache der normalen täglichen Ausscheidungsmenge von 0,4 bis 0,6 mg/24 h von gesunden Ratten. Figur 1 zeigt den Verlauf der Harnsäureausscheidung in Abhängigkeit von den verabreichten Dosen MDA/PI während der Behandlungszeit von 22 Tagen.

Nach 22 Behandlungstagen wurde die Ratte getötet, und die Autopsie ergab, daß im Bereich der linken Hüfte ein Tumor einer Größe von etwa 5 x 4 x 4 cm existierte, der sich bis zum Hüftknochen erstreckte. Die angeschnittene Tumoroberfläche war makroskopisch in einem Bereich von 3 x 2 x 2 cm aufgelöst und wies dort eine grau-weiße Farbe mit eingelagerten Zysten auf. Die eingehende mikroskopische Untersuchung dieses Tumorbereichs ergab, daß das Tumorgewebe in ödematöse Auflockerungen und Nekrosen überging, wobei sich insbesondere um die zystischen Strukturen umfangreiche Gewebsnekrosen feststellen ließen. Das äußere umgebende Tumorgewebe bestand aus spindeligen Zellen und Zellkernen sowie eingestreuten stark polymorphen Strukturen mit Riesenkernen und Chromatinverdichtungen.

Herz, Lunge, Leber und Hoden der Ratte wiesen keine Anomalitäten auf, abgesehen von einer venösen Hyperämie aufgrund der erhöhten Flüssigkeitsaufnahme und des einsetzenden Nierenversagens.

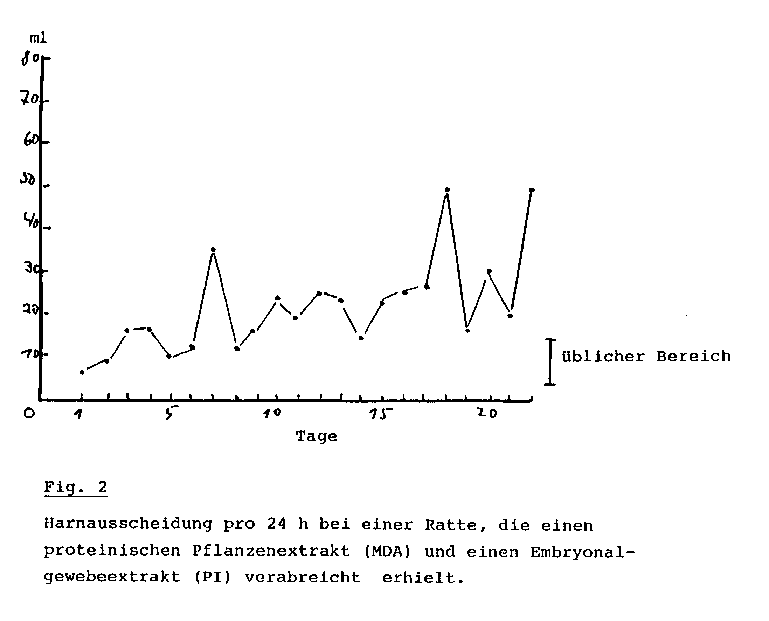

**Figur 2** zeigt in diagrammatischer Darstellung die Abhängigkeit der täglichen Urinausscheidung von den verabreichten Dosen MDA/PI (gleiche Ratte wie in Figur 1).

Die histologischen Befunde sowie die erhöhte Ausscheidung von Harnsäure im Urin beweisen die Zerstörung einer großen Menge von Tumorzellen des Fibrosarkoms.

### IV. Krankengschichte.

Eine 66jährige Frau wurde aufgrund eines progressiven Wachstums der linken Brustdrüse ins Krankenhaus überwiesen. Die Untersuchung ergab, daß links zwei vergrößerte, harte Achselknoten vorlagen. Am 4. Februar 1985 wurde die gesamte linke Brust zusammen mit einem elliptischen Hautbezirk, den Hauptbrustmuskeln und den linken Achsellymphknoten entfernt. Der Tumor hatte makroskopisch Faustgröße. Zum Schließen der Wunde mußte eine Hautverpflanzung vom Oberschenkel vorgenommen werden. Die Histopathologie zeigte ein invasives Gangkarzinom mit Metastasen in den Achselknoten. Zwei Monate nach der Operation führte der Lymphabfluß aus der Brust zu einer Vergrößerung der beiden linken supraklavikularen Lymphknoten, und es war zu einer ausgedehnten Metastasierung gekommen.

Der Patientin wurden daraufhin täglich 50 mg MDA verabreicht, begleitet von einer 15 Wochen fortgesetzten 2maligen Injektion von 24 mg PI in der Woche. Innerhalb von 2 Monaten verschwanden die Metastasen in der Oberschlüsselbeingrube. Bis März 1989 zeigte die Patientin keinerlei Zeichen eines Rückfalls, und zwar weder im klinischen Befund noch anhand der biochemischen Analyseergebnisse.

Es hat sich gezeigt, daß die Dosierung der beiden zusammen zu verabreichenden Substanzen MDA und PI in Abhängigkeit von der Art (Entwicklungszustand) und Masse des jeweiligen zu bekämpfenden Tumors in weiten Grenzen variiert werden kann.

Die Dosen beider Substanzen liegen jeweils im Bereich von 1 bis 10 mg/kg Körpergewicht. Beide Substanzen MDA und PI werden dabei in im wesentlichen gleichen Mengen verabreicht. Die physiologischen Wirkungen und die histologischen Befunde lassen eine Tumorauflösung (Nekrose mit Ödem) erkennen, begleitet von ein Hyperämie im geschädigten Organ. Die Wirkung betrifft selektiv nur die Zerstörung maligner Zellen, ohne daß gesunde Zellen erkennbar geschädigt werden.

Abschließend ist noch darauf hinzuweisen, daß die aus dem Photosyntheseapparat von Pflanzen gewonnene Proteinfraktion aus praktischen Gründen vorzugsweise aus solchen Pflanzen gewonnen wird, die keine giftigen oder anderweitig in unerwünschter Weise physiologisch aktiven Bestandteile enthalten, da die Anwesenheit derartiger Bestandteile infolge des Erfordernisses ihrer Abtrennung das Verfahren zur Gewinnung der gewünschten Proteinfraktion erschwert. Bevorzugt werden daher als Rohstoffe autotrophe Pflanzen verwendet, die normalerweise nicht zu den Heilpflanzen gezählt werden. Bei Verwendung derartiger Pflanzen kann es in günstigen Fällen ausreichen, die zerkleinerten Pflanzen ohne den Schritt einer Isolierung der Chloroplasten mit einer hypotonischen wäßrigen, sauren Alkohollösung zu behandeln.

Es ist ferner noch darauf hinzuweisen, daß die in der vorliegenden Anmeldung beschriebene Auftrennung des äthanolischen Extrakts durch trägerfreie Ablenkungselektrophores nur eines der möglichen Verfahren beschreibt und daß andere bekannte Abtrennungsverfahren (säulenchromatographische Verfahren unter Verwendung von Molekularsieben oder pH-Gradienten wie Gelfiltration oder Ionenaustauschchromatographie oder gegebenenfalls auch Affinitätschromatographie oder selektive Extraktionsverfahren) ebenfalls geeignet erscheinen und für eine industrielle Herstellung in größerem Maßstab sogar Vorteile aufweisen können.

## Patentansprüche

1. Proteinfraktion mit Sauerstoffaktivität aus dem Photosyntheseapparat von Pflanzen oder aus dessen Vorstufen, die erhältlich ist durch an sich bekannte Isolierung von Chloroplasten und/oder ihren Vorstufen aus autotrophen Pflanzen oder autotrophen niederen Lebewesen, Waschen der isolierten Chloroplastenfraktion, Behandeln der isolierten und gewaschenen Chloroplasten mit einer hypotonischen Äthanollösung, Abtrennen des Sediments, Auftrennen der alkoholischen Lösung durch trägerfreie Ablenkungselektrophorese und Gewinnung einer gelblichen, Sauerstoff freisetzenden Proteinfraktion sowie ggf. anschließendes Aufkonzentrieren dieser Fraktion durch Ultrafiltration, Dialyse des Ultrafiltrationsretentats gegen Wasser sowie Gefriertrocknen, zur Anwendung als in Kombination mit einem aus embryonalem Gewebe oder aus maternalem Uterusgewebe von Plazentatieren gewonnenen Proteingemisch per Injektion oder Infusion zu verabreichender pharmazeutischer Wirkstoff zur selektiven Auflösung von Krebszellen im lebenden Warmblüterorganismus.

2. Proteinfraktion nach Anspruch 1 zur Anwendung als per Injektion oder Infusion zu verabreichender pharmazeutischer Wirkstoff zur Nachbehandlung von Narben, Keloiden sowie von entzündlichen Prozessen, die auf die Einwirkung ionisierender Strahlung zurückgehen.

3. Proteinfraktion nach Anspruch 1 oder 2, dadurch gekennzeichnet, daß sie ganz oder überwiegend aus einer Proteinfraktion mit einem Molekulargewicht von 67000 +/- 10000 d (bestimmt durch SDS-Gradienten-Gelelektrophorese in Gegenwart eines Markers für den pH-Bereich von 4,7 bis 10,6) besteht.

4. Proteinfraktion nach Anspruch 1, 2 oder 3, dadurch gekennzeichnet, daß sie in Gegenwart von Protonenakzeptoren bei Lichtzutritt und Erwärmung bzw. unter IR-Komponenten enthaltendem Licht unter Freisetzung von Sauerstoff aus Wasser zersetzlich ist.

5. Proteinfraktion nach einem der Ansprüche 1 bis 4, dadurch gekennzeichnet, daß sie aus Equisetum arvense erhalten wurde.

6. Pharmazeutische Erzeugnisse, enthaltend (a) eine Proteinfraktion gemäß Anspruch 1 und (b) ein aus embryonalem Gewebe oder aus maternalem Uterusgewebe von Plazentatieren gewonnenes Proteingemisch als per Injektion oder Infusion im Gemisch oder in unmittelbarer zeitlicher Aufeinanderfolge zu verabreichendes Kombinationspräparat zur selektiven Auflösung von Krebszellen im lebenden Warmblüterorganismus.

7. Pharmazeutische Erzeugnisse nach Anspruch 6, dadurch gekennzeichnet, daß sie die Proteinfraktion (a) und das Proteingemisch (b) in gefriergetrockneter Form enthalten.

8. Pharmazeutische Erzeugnisse nach Anspruch 6 oder 7, dadurch gekennzeichnet, daß das Proteingemisch (b) erhältlich ist durch Zerkleinern tiefgefrorener Embryonen, Foeten, Plazenten, Nabelschnüren und/oder von maternalem Uterusgewebe von Plazentatieren unter sterilen Bedingungen in Gegenwart eines polaren wasserhaltigen organischen Lösungsmittels, Abtrennen sedimentierbarer Anteile aus der organischen Phase durch Sedimentation oder Sterilfiltration, Fein- und Mikrofiltration der von sedimentierbaren Anteilen befreiten organischen Lösung, Abfüllen des Filtrats ohne weitere Auftrennung in Proteinfraktionen sowie ggf. Gefriertrocknen, wobei alle genannten Verfahrensschritte unter sterilen Bedingungen und ausreichnder Kühlung, vorzugsweise in einem Laminar-Flow-System bei Temperaturen um +4°C, durchgeführt werden.

9. Pharmazeutische Erzeugnisse nach einem der Ansprüche 6 bis 8, dadurch gekennzeichnet, daß sowohl die Proteinfraktion (a) als auch das Proteingemisch (b) zusätzlich einem Reinigungsschritt zur Entfernung von Endotoxinen, vorzugsweise durch Adsorption an einem geeigneten Adsorptionsmittel, unterzogen wurden.

## Claims

1. Protein fraction which has oxygen activity and which is derived from the photosynthetic system of plants or from its precursors, which can be obtained by isolation of chloroplasts and/or their precursors from autotrophic plants or autotrophic lower organisms, which isolation is known per se, washing of the isolated chloroplast fraction, treating the isolated and washed chloroplasts with a hypotonic ethanol solution, removal of the sediment, resolution of the alcoholic solution by supportfree deflection electrophoresis and obtaining a yellowish, oxygen-liberating protein fraction, and, if appropriate, subsequent concentration of this fraction by ultrafiltration, dialysis of the ultrafiltration retentate against water and freeze-drying, for use as a pharmaceutical active substance to be administered by way of injection or infusion, in combination with a protein mixture derived from embryonic tissue or from maternal uterus tissue of placentals, for the selective dissolution of cancer cells in live organisms of warm-blooded species.

2. Protein fraction according to Claim 1 for use as a pharmaceutical active substance to be administered by way of injection or infusion, for the aftertreatment of scars, keloids and of inflammatory processes caused by the action of ionizing radiation.

3. Protein fraction according to Claim 1 or 2, characterized in that it consists entirely, or largely, of a protein fraction having a molecular weight of 67000 +/- 10000 d (determined by SDS gradient gel electrophoresis in the presence of a marker for the pH range of 4.7 to 10.6).

4. Protein fraction according to Claim 1, 2 or 3, characterized in that it decomposes in the presence of proton acceptors under the influence of light and heating or under light containing IR components, while liberating oxygen from water.

5. Protein fraction according to one of Claims 1 to 4, characterized in that it had been obtained from Equisetum arvense.

6. Pharmaceutical products containing (a) a protein fraction according to Claim 1 and (b) a protein mixture derived from embryonic tissue or maternal uterus tissue of placentals, as a preparation combination to be administered by way of injection or infusion, as a mixture or in immediate succession, for the selective dissolution of cancer cells in live organisms of warm-blooded species.

7. Pharmaceutical products according to Claim 6, characterized in that they contain the protein fraction (a) and the protein mixture (b) in freeze-dried form.

8. Pharmaceutical products according to Claim 6 or 7, characterized in that the protein mixture (b) can be obtained by comminuting deep-frozen embryos, fetuses, placentae, umbilical cords and/or maternal uterus tissue of placentals under sterile conditions in the presence of a polar water-containing organic solvent, removing components which are capable of separation from the organic phase by sedimantation or sterile filtration, fine filtration and microfiltration of the organic solution which has been freed from the components which are capable of separation, filling the filtrate into containers without further resolution into protein fractions and, if appropriate, freeze-drying, all the process steps mentioned being carried out under sterile conditions and sufficient cooling, preferably in a laminar flow system at temperatures around +4° C.

9. Pharmaceutical products according to one of Claims 6 to 8, characterized in that both the protein fraction (a) and the protein mixture (b) have been additionally subjected to a purification step for the removal of endotoxins, preferably by adsorption on a suitable adsorbent.

## Revendications

1. Fraction protéique à activité oxygène provenant de l'appareil photosynthétique de plantes ou de leurs précurseurs, qui peut être obtenue par isolation, en soi connue, de chloroplastes et/ou de leurs précurseurs provenant de plantes autotrophes ou d'organismes inférieurs autotrophes, lavage de la fraction isolée de chloroplastes, traitement des chloroplastes isolés et lavés avec une solution hypotonique d'éthanol, séparation du sédiment, séparation de la solution alcoolique par électrophorèse sélective sans support et récupération d'une fraction protéiquejaunâtre libérant de l'oxygène, ainsi qu'éventuellement concentration ultérieure de cette fraction par ultrafiltration, dialyse à l'eau de la fraction retenue par ultrafiltration, ainsi que lyophilisation, pour utilisation comme produit pharmaceutique actif, à administrer par injection ou par infusion, en combinaison avec un mélange de protéines extrait de tissus embryonnaires ou de tissus de l'utérus maternel d'animaux à placenta, pour la solubilisation sélective de cellules cancéreuses dans des organismes vivants à sang chaud.

2. Fraction protéique selon la revendication 1, pour utilisation comme produit actif pharmaceutique à administrer par injection ou infusion, pour le traitement de cicatrices, de chéloïdes ainsi que de processus inflammatoires provoqués par l'action de rayonnement ionisant.

3. Fraction protéique selon la revendication 1 ou 2, caractérisée en ce qu'elle est constituée totalement ou principalement d'une fraction protéique d'un poids moléculaire de 67.000 +/- 10.000 (défini par électrophorèse sur gel à gradient SDS en présence d'un marqueur, dans la plage de pH de 4,7 à 10,6).

4. Fraction protéique selon la revendication 1, 2 ou 3, caractérisée en ce qu'elle se décompose avec libération d'oxygène de l'eau en présence d'accepteurs de protons, par exposition à la lumière et chauffage ou par exposition à une lumière contenant des composantes IR.

5. Fraction protéique selon l'une des revendications 1 à 4, caractérisée en ce qu'elle a été obtenue à partir d'Equisetum Arvense.

6. Produits pharmaceutiques contenant (a) une fraction protéique selon la revendication 1 et (b) un mélange de protéines extrait d'un tissu embryonnaire ou d'un tissu de l'utérus maternal d'animaux à placenta, comme préparation combinée à administrer par injection ou infusion, en mélange ou en succession immédiate, en vue de la mise en solution sélective de cellules cancéreuses dans l'organisme vivant à sang chaud.

7. Produits pharmaceutiques selon la revendication 6, caractérisés en ce qu'ils contiennent la fraction protéique (a) et le mélange de protéines (b) sous forme lyophilisée.

8. Produits pharmaceutiques selon la revendication 6 ou 7, caractérisés en ce que le mélange de protéines (b) peut être obtenu par broyage d'embryons, foetus, placentas, cordons ombilicaux et/ou tissus de l'utérus maternel d'animaux à placenta, congelés, en conditions stériles et en présence d'un solvant organique polaire contenant de l'eau, extraction des fractions sédimentables hors de la phase organique par sédimentation ou filtration stérile, filtration fine et micro-filtration de la solution organique débarrassée des fractions sédimentables, extraction du filtrat sans séparation supplémentaire en fractions protéiques ainsi qu'éventuellement lyophilisation, toutes lesdites étapes de procédé étant effectuées dans des conditions stériles et sous refroidissement suffisant, par exemple dans un système à écoulement laminaire, à des températures autour de +4°C.

9. Produits pharmaceutiques selon l'une des revendications 6 à 8, caractérisés en ce qu'aussi bien la fraction protéique (a) que le mélange de protéines (b) sont soumis en supplément à une étape de purification pour extraction d'endotoxines, de préférence par adsorption sur un agent d'adsorption approprié.
